# EUROPEAN PATENT APPLICATION

(11) **EP 1 498 392 A2**
(43) Date of publication of application: **19.01.2005**
(21) Application number: 04103305.1
(22) Date of filing: 12.07.2004
(51) Int. Cl.: C02F 9/00, C02F 1/66, A61K 35/08

(54) **Device and method for treating drinking water for topical detergent, cosmetic and/or therapeutic use**

(30) Priority: 11.07.2003 IT MI20031433
(71) Applicant: DERMING S.r.l., 20052 MONZA (IT)
(72) Inventor: Sparavigna, Adele, 20052 Monza (IT); Setaro, Michele, 20052 Monza (IT)
(74) Representative: Jorio, Paolo, Dr. Ing.

(57) **Abstract**

A device (1) for treating drinking water to produce water for topical detergent, cosmetic and/or therapeutic use has a hydraulic circuit (3) in which flows a water stream (4) drawn from a drinking water mains (8); and treatment units (16) are arranged along the circuit to alter the chemical-physical properties, in particular the pH, hardness, and chlorine content, of the water stream.

## Description

The present invention relates to a device and method for treating drinking water to produce water for topical detergent, cosmetic and/or therapeutic use.

Drinking water is water which, drawn from various sources and appropriately treated, is suitable for human consumption, is mainly supplied by water mains (i.e. facilities and equipments for water intake, piping and distribution), and, to be classified "drinkable" and distributed to users, must conform with strict legal requirements.

To meet such requirements, drinking water normally has a substantially neutral pH, is relatively hard (i.e. relatively rich in so-called "scaling" salts, in particular calcium bicarbonate and magnesium), and has a relatively high chlorine content (added as a disinfectant).

In view of the above characteristics, drinking water is not altogether satisfactory when used, together with (surface-active) detergents, for simply cleansing human skin, and even less so when used for cosmetic or therapeutic treatment.

In fact, to begin with, the pH of human skin is acid, whereas that of drinking water is substantially neutral, so that drinking water has an aggressive effect on the skin when applied even simply for washing or rinsing purposes.

Moreover, in the presence of dermatological diseases or various pathologies, the pH of the skin is known to be altered, and is normally more basic than that of healthy skin. For example, the skin of subjects suffering from atopic dermatitis (constitutional eczema) has a more basic pH than that of normal subjects, both in the skin areas affected by the eczema, and, to a lesser but still significant degree, in the apparently healthy skin areas.

In these conditions, applying drinking water with a neutral pH obviously further aggravates the already abnormal condition of the skin.

Moreover, being hard, drinking water calls for a relatively large amount of detergent which is invariably aggressive to some extent and therefore harmful on both healthy and, above all, abnormal skin.

Finally, various substances in drinking water (particularly chlorine) have an aggressive, and therefore, harmful effect on the skin.

Any type of dermatological treatment (cleansing, cosmetic or therapeutic) may call for rinsing the skin afterwards, and, precisely on account of the above characteristics, rinsing the skin with drinking water may possibly impair any benefits derived from the treatment.

The above problems apply to both domestic and professional use, e.g. beauty centres, hospitals, clinics, etc.

It is therefore an object of the present invention to solve the above problems by providing a device and method for treating drinking water, which provide, in a straightforward, practical, low-cost manner, for producing water suitable for detergent, cosmetic and/or therapeutic use.

According to the present invention, there are provided a device and method for treating drinking water to produce water for topical detergent, cosmetic and/or therapeutic use, as claimed in the accompanying Claims 1 and 10 respectively.

According to the present invention, there is also provided a method of cosmetically treating human skin, as claimed in the accompanying Claim 11.

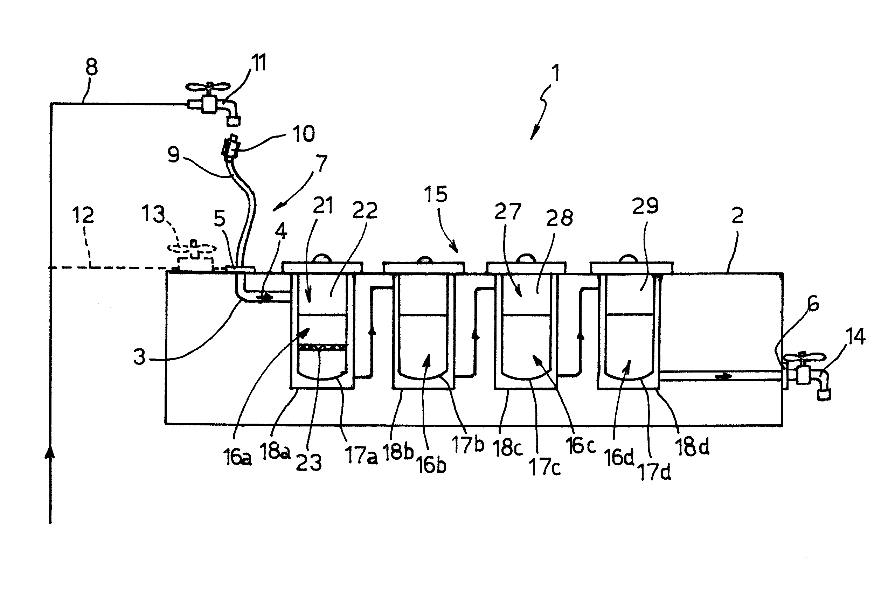

A non-limiting embodiment of the invention will be described, purely by way of example, with reference to the accompanying drawing, which shows, schematically, a device in accordance with the invention.

Number 1 in the accompanying drawing indicates as a whole a device for treating drinking water to produce water for topical detergent, cosmetic and/or therapeutic use.

Device 1 comprises a casing 2 housing a hydraulic circuit 3, along which flows a water stream, indicated by arrow 4 in the drawing, and which has an inlet 5 and an outlet 6 located at opposite ends of casing 2. Inlet 5 has connecting means 7 for connection to a drinking water mains 8; connecting means 7 comprise, for example, a hose 9 fitted on its free end with a fitting 10 connectable in any known manner to an end fixture 11 of mains 8; and end fixture 11 may conveniently be a tap of an existing sanitary fixture (bathtub or basin), or a tap installed specifically for use with device 1.

Alternatively, as shown by the dash line in the drawing, device 1 may be connected directly to mains 8, in which case, connecting means 7 comprise a branch pipe 12 from mains 8, and a tap 13 or other suitable control member for feeding water stream 4 into circuit 3.

Outlet 6 is also provided with an optional tap 14.

Device 1 comprises treating means 15 for altering the chemical-physical properties of water stream 4 flowing in circuit 3.

Treating means 15 comprise a number of treating units 16 arranged in series along circuit 3 to intercept water stream 4.

In the non-limiting example shown, units 16 comprise respective replaceable cartridges 17 inserted removably inside respective seats 18 in casing 2, and arranged in series along circuit 3 to intercept water stream 4.

Each unit 16, i.e. each cartridge 17, provides for a specific treatment of water stream 4.

More specifically, a first unit 16a has water softening means 21. For example, unit 16a comprises a softening cartridge 17a housed in a seat 18a, and having a dispenser 22 (of any known type and only shown schematically in the drawing) for dispensing substances by which to precipitate calcium and other salts dissolved in the water, and a precipitate filter 23.

Calcium may be removed or reduced, for example, using known chemical precipitation systems employing lime, sodium carbonate or cation exchange resins.

A second unit 16b is a chlorine-removal unit comprising a chlorine-removal cartridge 17b housed in a seat 18b.

Chlorine may be removed using any known system, such as known polycarbonate lines.

A third unit 16c has pH-adjusting means 27 for imparting an acid pH to water stream 4, and specifically a pH ranging between approximately 3.0 and 6.0, and preferably of about 5.5.

For example, unit 16c comprises a pH-regulating cartridge 17c housed in a seat 18c, and having a known dispenser 28 (shown only schematically in the drawing) for releasing into water stream 4 an acid substance by which to form a buffer system.

Examples of suitable buffer systems are citric/citrate acid; carbonic/bicarbonate acid; lactic/lactate acid.

Advantageously, a number of pH-regulating cartridges 17c are provided, which can be inserted selectively inside seat 18c, and each of which provides for producing a predetermined pH value of water stream 4.

A fourth unit 16d is an enriching unit, which comprises an enriching cartridge 17d housed in a seat 18d, and having a known dispenser 29 for releasing into water stream 4 one or more substances selected according to the specific cosmetic or therapeutic treatment to be performed.

Advantageously, a number of enriching cartridges 17d are provided, which can be inserted selectively inside seat 18d, and each of which provides for releasing one or more substances for treating a specific pathology or disorder.

In normal conditions, water stream 4 is circulated in device 1 by the operating pressure of mains 8, though device 1 may comprise circulating means of its own, e.g. a known pump (not shown) for circulating water between inlet 5 and outlet 6.

The order in which units 16 are arranged along circuit 3 may, obviously, be other than as shown purely by way of example.

Device 1 implementing the method according to the invention operates as follows.

When water is required for topical detergent, cosmetic and/or therapeutic use, the user activates circulation of water stream 4, drawn from mains 8, through device 1.

Device 1 alters the chemical-physical properties of water stream 4. More specifically, a water-softening step is performed in unit 16a; a chlorine-removal step is performed in unit 16b; a pH-adjusting step is performed in unit 16c, in which the pH of water stream 4 is brought to an acid pH as described above and of a specific value depending on the selected pH-regulating cartridge 17c; and an enriching step is performed in unit 16d, in which water stream 4 is enriched with one or more substances released by cartridge 17d.

Obviously, the above steps may be performed in other than the order described, depending on the arrangement of respective units 16 along circuit 3. Some or all of the steps indicated may be performed simultaneously. For which purpose, provision may be made, for example, for an integrated unit incorporating the functions of a number of units 16, i.e. a multifunction cartridge which, inserted inside a seat 18, performs a number of different functions; or a number of cartridges 17 may be housed in the same seat 18, in which the respective functions are performed simultaneously.

Whichever the case, the treated water issuing from outlet 6 can be applied directly to the human skin for detergent, cosmetic and therapeutic purposes, and for preventing skin disorders, flaws and pathologies.

By means of the method and device according to the invention, water can therefore be produced cheaply and easily for effective use as a straightforward skin cleanser, for cosmetic and therapeutic treatment, and for preventing the onset of skin pathologies.

The device and method according to the invention enable this to be done using ordinary drinking water (which is obviously available cheaply to any user connected to a public water mains) by eliminating the aforementioned drawbacks typically associated with untreated drinking water.

The water prepared according to the invention, in fact, has a pH similar to that of human skin, so does not even temporarily affect the pH of the skin, and in fact even enhances its buffer capacity; is relatively soft, and therefore provides for effective cleansing with a small amount of surface-active detergent; and contains substantially no chlorine or other aggressive agents. In short, the water produced according to the invention greatly reduces the aggressive action on the skin of normal drinking water.

The water treated in accordance with the invention is therefore ideal for rinsing the skin after any type of dermatological (cleansing, cosmetic or therapeutic) treatment, and in no way impairs the benefits derived from the treatment.

Moreover, the device according to the invention is cheap and easy to produce, install and operate, and is designed for both domestic and professional use (beauty centres, hospitals, clinics, etc.).

Even subjects with healthy or normal skin, but especially those suffering from various skin disorders or pathologies, may benefit from the advantages of the present invention.

Precisely on account of significantly reducing skin aggression, the water prepared using the device and method according to the invention safeguards the skin against harm of any sort, and, if used regularly, provides for effectively preventing the onset of various skin disorders or pathologies, or even straightforward skin defects.

The water prepared using the device and method according to the invention has proved particularly effective in treating all pathologies and disorders involving abnormal pH of the skin, such as atopic dermatitis (constitutional eczema) and contagious dermatitis (topical eczema).

Good results have also been obtained in the treatment of psoriasis, ichthyosis simplex, various skin infections, nappy rash, and dermatitis typically associated with the elderly.

Application of the water produced in accordance with the invention has also proved effective in diabetics, hemiplegics, and haemodialysis subjects.

As shown, depending on the pathology of the user, the device according to the invention provides for targeting water treatment by selecting the appropriate cartridges from those available: in particular, water can be produced with a specific pH value selected according to the condition of the subject for treatment; and substances beneficial to a specific disorder can be added to the water.

## Claims

1. A device (1) for treating drinking water to produce water for topical detergent, cosmetic and/or therapeutic use, comprising a hydraulic circuit (3) having an inlet (5) with connecting means (7) for connection to a drinking water mains (8), and an outlet (6) for the treated water; and treating means (15) for altering the chemical-physical properties of a water stream flowing in the circuit; the device being **characterized in that** said treating means (15) comprise pH-adjusting means (27).

2. A device as claimed in Claim 1, **characterized in that** the pH-adjusting means (27) impart an acid pH to said water stream (4).

3. A device as claimed in Claim 2, **characterized in that** the pH-adjusting means (27) comprise a first dispenser (28) for releasing into said water stream (4) an acid substance by which to form a buffer system.

4. A device as claimed in one of the foregoing Claims, **characterized in that** the treating means (15) comprise water softening means (21).

5. A device as claimed in one of the foregoing Claims, **characterized in that** the treating means (15) comprise a unit (16b) for removing chlorine dissolved in said water stream (4).

6. A device as claimed in one of the foregoing Claims, **characterized in that** the treating means (15) comprise an enriching unit (16d) for releasing one or more substances into said water stream (4).

7. A device as claimed in one of the foregoing Claims, **characterized in that** the treating means (15) comprise at least one treatment unit (16) comprising a replaceable cartridge (17) inserted removably inside a seat (18) along the circuit (3) to intercept the water stream (4).

8. A device as claimed in Claim 7, **characterized by** comprising a number of cartridges (17) selectively insertable inside said seat (18); each cartridge performing a specific treatment of the water stream (4).

9. A device as claimed in one of the foregoing Claims, **characterized in that** the treating means (15) comprise a number of treatment units (16) arranged in series along the circuit (3) to intercept the water stream (4), and each of which performs a specific treatment of the water stream (4).

10. A method of treating drinking water to produce water for topical detergent, cosmetic and/or therapeutic use, and comprising the steps of drawing a water stream (4) from a drinking water mains (8), and treating the water stream to alter the chemical-physical properties of said water stream; the method being **characterized by** comprising the step of adjusting the pH of said water stream.

11. A method of cosmetically treating human skin, and comprising the steps of drawing a water stream (4) from a drinking water mains (8), and treating the water stream to alter the chemical-physical properties of said water stream; the method being **characterized by** comprising the steps of adjusting the pH of said water stream, and applying the treated water to the human skin.

12. A method as claimed in Claim 10 or 11, **characterized in that**, at said step of adjusting the pH, an acid pH is imparted to said water stream.

13. A method as claimed in Claim 12, **characterized in that**, at said step of adjusting the pH, an acid substance is released into said water stream to form a buffer system.

14. A method as claimed in one of Claims 10 to 13, **characterized by** comprising a water softening step.

15. A method as claimed in one of Claims 10 to 14, **characterized by** comprising a step of removing chlorine dissolved in said water stream.

16. A method as claimed in one of Claims 10 to 15, **characterized by** comprising a step of releasing one or more substances into said water stream.

17. A method as claimed in one of Claims 10 to 16, **characterized in that** the treatment step is performed by at least one treatment unit (16) comprising a replaceable cartridge (17) inserted removably inside a seat (18) to intercept said water stream (4).

18. A method as claimed in Claim 17, **characterized by** comprising the steps of providing a number of cartridges (17) selectively insertable inside said seat (18), each cartridge performing a specific treatment of the water stream (4); and selecting the cartridge suitable for a specific treatment.
